# EUROPÄISCHE PATENTSCHRIFT

(11) **EP 2 564 861 B1**
(45) Veröffentlichungstag und Bekanntmachung des Hinweises auf die Patenterteilung: **17.06.2015**
(21) Anmeldenummer: 12181044.4
(22) Anmeldetag: 20.08.2012
(51) Int. Cl.: A61K 36/38, A61K 9/00, A61P 31/04

(54) **Pharmazeutische Formulierung zur Behandlung von Mastitis bei Säugern**
Pharmaceutical formula for treating mastitis in mammals
Formule pharmaceutique de traitement de la mastite chez les mammifères

(30) Priorität: 01.09.2011 DE 102011081943
(43) Veröffentlichungstag der Anmeldung: 06.03.2013
(73) Patentinhaber: Walter RAU Neusser Öl und Fett AG, 41460 Neuss (DE)
(72) Erfinder: Brinkmann, Bernd, 41812 Erkelenz (DE); Elsser-Gravesen, Dr. Ing. Dieter, 8240 Risskov (DK)
(74) Vertreter: Bauer, Dirk

(56) Entgegenhaltungen:
- FR-A1- 2 939 676
- KAITHWAS GAURAV ET AL: "Linum usitatissimum (linseed/flaxseed) fixed oil: antimicrobial activity and efficacy in bovine mastitis", INFLAMMOPHARMACOLOGY, Bd. 19, Nr. 1, Februar 2011 (2011-02), Seiten 45-52, XP002688296, ISSN: 0925-4692
- RAMAN B VENKATA ET AL: "Antibacterial Activities of Some Folk Medicinal Plants of Eastern Ghats", JOURNAL OF PURE AND APPLIED MICROBIOLOGY, Bd. 3, Nr. 1, April 2009 (2009-04), Seiten 187-194, XP009165098, ISSN: 0973-7510
- Kilham, Ch.: "Tamanu Oil. A tropical topical remedy", Herbalgram, Bd. 63 2004, Seite 10, XP002688297, Gefunden im Internet: URL:http://www.advancedhealing.com/uploads /Tamanu_Oil.pdf [gefunden am 2012-11-29]

## Beschreibung

### Einleitung

Die Erfindung betrifft die Verwendung einer pharmazeutischen Formulierung zur Behandlung von Mastitis bei landwirtschaftlichen Nutztieren.

Ferner offenbart die Erfindung eine pharmazeutische Formulierung enthaltend Calophyllum Inophyllum Samenöl und eine natürliche Fettzubereitung.

### Stand der Technik

Bei der Mastitis handelt es sich um eine Entzündung der Brustdrüse beziehungsweise der Milchdrüse bei Säugern. Beispielsweise in Form der so genannten *Mastitis puerperalis* tritt sie beim Menschen üblicherweise während der Stillzeit durch die Infektion der Milchdrüse der weiblichen Brust mit Bakterien der Spezies *Staphylococcus aureus* auf, die sich unter anderem vermehrt in der Mundhöhle des Menschen und somit auch der eines Kindes findet. Während des Stillens kann es schließlich zur Infektion der Milchdrüse der Mutter kommen. Begünstigt wird dieser Vorgang durch lange Verweilzeiten der Milch in der Milchdrüse und einen dadurch entstehenden Milchstau. Dies kann speziell im Falle langer Intervalle zwischen den Stillzeiten auftreten.

Neben der Bedeutung für den Menschen ist vor allem die Behandlung von Mastiden bei landwirtschaftlichen Nutztieren von allgemeinem Interesse, da der wirtschaftliche Schaden durch Mastiden bei Tieren, die zur Milchproduktion genutzt werden, erheblich ist. Beispielsweise tritt die Mastitis häufig bei Milchkühen zutage, wobei die Infektion unter anderem auf Streptokokken der Spezies *Streptococcus agalactiae* und *Streptococcus dysgalactiae* sowie - wie im Fall der *Mastitis puerperalis -* auf Staphylokokken der Spezies *Staphylococcus aureus* zurückzuführen ist. Hierbei ist zu sagen, dass die Bedeutung der Infektion durch *Staphylococcus aureus* an Bedeutung zunimmt, da diese Spezies inzwischen besonders umfangreiche Resistenzen gegen Antibiotika entwickelt hat und nur noch schwer behandelbar ist. *Streptococcus agalactiae* hingegen spricht auf die Behandlung mit Antibiotika gut an und konnte in der Vergangenheit entsprechend stark dezimiert werden.

Infolge der Erkrankung der Tiere mit Mastitis kommt es zu Ausfällen der Milchproduktion sowie zu Behandlungskosten und gegebenenfalls sogar zu einem Aussondern eines jeweilig betroffenen Tieres. Der wirtschaftliche Schaden für Milchbauern durch diese Vorfälle ist beträchtlich und wird beispielsweise in Deutschland mit ca. 255 Mio. Euro pro Jahr beziffert.

Die Behandlung von Mastiden sieht heutzutage üblicherweise den Einsatz von Antibiotika vor. Wie bereits erwähnt führt diese Behandlung jedoch nicht immer zum Erfolg, da - beispielsweise bei Milchkühen - die Bakterien effektive Überlebensstrategien gegen Antibiotika entwickelt haben. Besonders im Hinblick auf die Ausbildung von weiteren Resistenzen der Bakterien ist man daher heutzutage bestrebt, den Einsatz von Antibiotika zu reduzieren. Der in der Vergangenheit häufig praktizierte und manchmal unüberlegte Einsatz von Antibiotika ist entsprechend nicht länger die erste Wahl wenn eine Mastitis diagnostiziert wurde. Als problematisch ist hierbei zu beurteilen, dass alternative Behandlungsmethoden rar sind. Zwar existieren homöopathische Therapien, deren Erfolgsquoten sind jedoch als eher gering einzustufen.

Die Motivation, die Verwendung von Antibiotika zu reduzieren, ist ferner durch den Umstand begründet, dass durch die verbreitete Anwendung von Antibiotika zur Behandlung von erkrankten Tieren eine messbare Anreicherung erstgenannter im Nahrungskreislauf stattfindet, so dass letztendlich der Mensch im Zuge der Nahrungsaufnahme zunehmend Antibiotika aufnimmt, wodurch die Bildung von Resistenzen einzelner Bakterienstämme weiter begünstigt wird. Diese Umstände veranlassen die Forschung zunehmend zu der Suche nach Wirkstoffen, die vergleichbar zu einem Antibiotikum antibakteriell wirken und in spezifischen Anwendungsbereichen zur Behandlung von Infektionskrankheiten - wie beispielsweise der Mastitis - eingesetzt werden können, indem sie die jeweilig verantwortlichen Bakterien abtöten oder zumindest deren Wachstum hemmen.

### Aufgabe

Der vorliegenden Erfindung liegt entsprechend die Aufgabe zugrunde, einen Wirkstoff sowie eine pharmazeutische Formulierung zur Verfügung zu stellen, mittels derer eine Behandlung von Mastitis bei landwirtschaftlichen Nutztieren durchgeführt werden kann, ohne dass ein Einsatz von Antibiotika nötig ist.

### Lösung

Die zugrunde liegende Aufgabe wird erfindungsgemäß dadurch gelöst, dass Calophyllum Inophyllum Samenöl als Wirkstoff einer pharmazeutischen Formulierung zur Behanldung landwirtschaftlicher Nutztiere zur Anwendung kommt.

In Untersuchungen des Wirkstoffs Calophyllum Inophyllum Samenöl - auch "Undi-Öl" oder "Tamanu-Öl" genannt - hat sich eine antimikrobielle Wirkung desselben gezeigt. Insbesondere konnte die wachstumshemmende beziehungsweise abtötende Wirkung des Undi-Öls gegen pathogene Staphylokokken festgestellt werden, wobei speziell eine gute Wirksamkeit gegen Staphylokokken der Spezies *Staphylococcus aureus* hervorzuheben ist. Die Untersuchungen hierzu wurden getrennt an humanpathogenen sowie an tierpathogenen *Staphylococcus au*reus-Stämmen durchgeführt, wobei stets Koagulase-positive und Koagulase-negative Stämme beteiligt waren. Die Koagulase-negativen Stämme sind dabei der "neutralen" Hautflora zuzuordnen und werden eher als opportunistisch im Zusammenhang mit einer Infektion eingestuft, während die Koagulase-positiven Stämme den pathogenen Stämmen zugerechnet werden können.

Im Rahmen der Durchführung von Hemmtests auf einer Nährmedium-Oberfläche konnte festgestellt werden, dass der Wirkstoff des Calophyllum Inophyllum Samenöls die pathogenen Staphylokokken entweder vollständig oder zumindest in besonderem Maße an ihrem Wachstum zu hemmen vermag. Die Koagulase-negativen Stämme, die aufgrund ihrer fehlenden Koagulasebildung allenfalls opportunistisch an dem Infektionsgeschehen teilnehmen, wurden durch das Undi-Öl in geringerem Maße an ihrem Wachstum gehindert, als dies bei den pathogenen Stämmen (Koagulase-positive Stämme) der Fall war.

Zur Beurteilung der bakteriziden Wirkung des Calophyllum Inophyllum Samenöls wurden ferner Anwendungen auf Oberflächen getestet. Diese Tests ergaben eine hohe Wirksamkeit gegen alle getesteten pathogenen *Staphylococcus aureus-*Stämme sowie eine deutliche Reduktion der Koagulase-negativen Staphylokokken sowie der Spezies *Staphylococcus saprophyticus* und *Staphylococcus epidermis.* Die Wirkung des Calophyllum Inophyllum Samenöls gegen pathogene sowie opportunistische Staphylokokken Stämme konnte also gezeigt werden.

Als besonderer Vorteil ist ferner hervorzuheben, dass das Undi-Öl gegen Milchsäurebakterien und Mikrokokken keine Wirkung entfaltet. Versuchsreihen haben zeigen können, dass derartige Bakterien, beispielsweise *Micrococcus luteus, Lactobacillus casei, Lactobacillus sakei* und *Lactobacillus plantarum,* von dem Calophyllum Inophyllum Samenöl vollständig unbehelligt blieben. Die "erwünschte" Hautflora auf der Hautoberfläche beispielsweise einer Milchkuh wird entsprechend durch den Wirkstoff weder in ihrem Wachstum gestört, noch durch ihn abgetötet. Diese selektive Wirkung des Calophyllum Inophyllum ermöglicht die Anwendbarkeit des Wirkstoffs auf Hautoberflächen landwirtschaftlicher Nutztiere und im Rahmen von Wundbehandlungen und verschafft ihm so ein breites Anwendungsspektrum.

Die erfindungsgemäße Verwendung der pharmazeutischen Formulierung im Rahmen der Behandlung von Mastitis, die - wie vorstehend erläutert - häufig auf Bakterien der Spezies *Staphylococcus aureus* zurückzuführen ist, schafft somit die Möglichkeit, die bereits genannten Nachteile einer Behandlung von Mastitis unter Verwendung von Antibiotika in der Nutztierhaltung wie gewünscht zu umgehen. Das erläuterte selektive Wirkungsspektrum des Wirkstoffs Calophyllum Inophyllum ermöglicht ferner die praktische Anwendung beispielsweise auf Hautoberflächen, ohne dass unschädliche Bakterien, die eine gesunde Hautflora ausmachen, angegriffen werden.

Als besonderer Vorteil der erfindungsgemäßen Verwendung einer pharmazeutischen Formulierung mit dem Wirkstoff des Calophyllum Inophyllum Samenöls sind die nachfolgenden Punkte zusammenfassend aufzuzählen:
- Hohe Wirksamkeit gegen pathogene Bakterien der Spezies *Staphylococcus aureus,* die Mehrheitlich für Mastitis verantwortlich sind
- Gute Wirksamkeit außerdem gegen opportunistische (Koagulase-negative) *Staphylococ*cus Stämme
- Selektive Wirksamkeit und somit keine negative Wirkung gegen Bakterien der erwünschten Hautflora, insbesondere gegen Mikrokokken und Milchsäurebakterien

Bisher war die Verwendung von Calophyllum Inophyllum lediglich aus anderen medizinischen Bereichen bekannt. Somit beschreibt beispielsweise die MX 2007 012225 A die Verwendung einer Zusammensetzung verschiedener Öle (unter anderem Calophyllum Inophyllum Samenöl) zur Behandlung von Hautkrankheiten bei Säugetieren, insbesondere bei Menschen. Calophyllum Inophyllum kommt hierbei zu einem relativ geringen Anteil zum Einsatz. In besagter Schrift wird der Anteil in der Zusammensetzung mit zwischen 3 % und 12 % angegeben.

Ein anderes Anwendungsgebiet beschreibt die CN 101263222 A, die die Geruchsbildung beim Menschen infolge Schwitzens behandelt. Somit wird zur Behandlung der im Achselbereich des Menschen üblicherweise vorhandenen Bakterienflora, welche zu großen Teilen aus Staphylokokken und Corynebakterien besteht, unter anderem der Einsatz von Calophyllum Inophyllum vorgeschlagen, wobei dies wiederum in eine Zusammensetzung mit anderen Wirkstoffen eingebettet ist.

Auf einem ähnlichen Gebiet bewegt sich die DE 10 2007 028 506 A1, die sich mit dem Themengebiet der Deodorantien beziehungsweise Antitranspirantien beschäftigt. Im Zusammenhang mit einer Vielzahl von antioxidativ wirkenden Pflanzenextrakten sowie antimikrobiell wirkenden Extrakten wird unter anderem Calophyllum Inophyllum genannt.

Auch Anwendungen im kosmetischen Bereich sind in der Literatur auffindbar. Somit beschreiben beispielsweise die Schriften FR 2942961 A1 und FR 2900047 A1 die Verwendung von Tamanuöl in Verbindung mit kosmetischen Zusammensetzungen, wobei in erstgenannter Schrift der Wirkstoff Calophyllum Inophyllum als narbenbildendes Mittel und in letztgenannter Schrift als Bestandteil einer Zusammensetzung zur Behandlung von fettiger Haut genannt sind.

Ebenfalls in seiner Wirkung als narbenbildendes Mittel wird Calophyllum Inophyllum Samenöl in der FR 2929121 A1 vorgeschlagen, welche sich mit der Pflege beziehungsweise der Reinigung der Haut - und hier insbesondere der Haut von Füßen - von Diabetes Patienten beschäftigt. Ähnlich hierzu bewegt sich außerdem die FR 2939676 A1 auf dem Gebiet der Pflege der Haut von Füßen von Diabetikern. Auch in diesem Fall soll Tamanuöl als Wirkstoff zur Narbenbildung verwendet werden, wobei die Anmeldung eine Zusammensetzung beschreibt, die eine Glycosidverbindung enthalten soll und zur Bekämpfung von Mikroorganismen, insbesondere solcher vom Typ *Staphylococcus aureus,* geeignet sein soll.

Im Hinblick auf die Behandlung von Haut beziehungsweise Hautkrankheiten wird Calophyllum Inophyllum des Weiteren im Rahmen der EP 2 218 447 A1 verwendet, die sich im Speziellen mit der antibakteriellen Wirkung von Silberionen auseinandersetzt und unter anderem auf den häufig in Verbindung mit Hautkrankheiten auftretenden Erreger *Staphylococcus aureus* eingeht. Das Calophyllum Inophyllum wird in diesem Zusammenhang als einer von mehreren möglichen Zusatzstoffen aufgeführt, der diese antibakterielle Wirkung der Silberionen unterstützen beziehungsweise verstärken soll.

Wie vorstehend bereits ausgeführt wurde wird Undi-Öl über diese nach dem Stand der Technik bekannten Anwendungen hinaus erfindungsgemäß zur Verwendung als Wirkstoff einer pharmazeutischen Formulierung bei der der Behandlung von Mastitis bei landwirtschaftlichen Nutztieren - und hier in erster Linie Milchkühen - vorgeschlagen. Dies hängt in erster Linie damit zusammen, dass im Vergleich zu der nach dem Stand der Technik üblichen Behandlungsmethodik unter Anwendung von Antibiotika sich neben der Vermeidung des weiteren Resistenzenaufbaus pathogener Bakterien sowie der generellen Unwirksamkeit gegenüber selbigen aufgrund bereits existierender Resistenzen ferner der besondere Vorteil des geringen Milchausschusses ergibt. Während Behandlungen mit Antibiotika dazu führen, dass die produzierte Milch sowohl während der Behandlung als auch in einem gewissen Zeitraum im Nachgang der Behandlung vernichtet werden muss, da diese mit den jeweiligen Antibiotika angereichert ist, bietet die erfindungsgemäße Verwendung einer pharmazeutischen Formulierung mit dem Wirkstoff des Calophyllum Inophyllum Samenöls den Vorteil, dass sogar parallel zur Behandlung der Mastitis die vom Nutztier gegebene Milch verwertet werden kann. Ein Ausschuss entfällt und damit einhergehend auch der entsprechende wirtschaftliche Schaden. Die erfindungsgemäße pharmazeutischen Formulierung ist dann besonders einfach zu verwenden, wenn sie in Form eines Öls, einer Creme, einer Salbe oder einer Emulsion vorliegt. Auf diese Weise kann eine Oberflächenbehandlung beispielsweise einer Hautoberfläche besonders einfach durch "Einreiben" durchgeführt werden, wobei der in der pharmazeutischen Formulierung enthaltene Wirkstoff Calophyllum Inophyllum direkt auf die mit den pathogenen Staphylokokken befallenen Hautpartien wirken kann. Da das Calophyllum Inophyllum die vorstehend erläuterte selektive Wirksamkeit aufweist, das heißt, dass es die zur erwünschten Hautflora gehörenden Milchsäurebakterien sowie Mikrokokken nicht angreift, ist die direkte Anwendung auf der Haut besonders einfach und ohne Nachteile durchführbar.

Die erfindungsgemäße pharmazeutischen Formulierung zur anspruchsgemäßen Verwendung ist dabei nicht auf eine Form der Mastitis hinsichtlich des auslösenden Bakteriums beschränkt, sondern kann vielmehr generell bei der Behandlung einer jeden Mastitis zum Einsatz kommen, insbesondere bei einer solchen, die durch bakterielle Infektionen mit Staphylokokken und/oder Streptokokken und/oder Klebsiella und/oder Actinomyces und/oder Corynebaktieren und/oder Listerien und/oder coliformen Erregern und/oder Pseudomonas hervorgerufen worden ist. Wie bereits vorstehend erläutert, stellt dabei die Infektion durch Staphylokokken die hauptsächliche Ursache für die Entstehung der Mastitis dar, wobei die Wirksamkeit der pharmazeutischen Formulierung gegen diese Spezies als besonders hoch einzustufen ist. Die erfindungsgemäße Verwendung auch in solchen Fällen, in denen die Infektion durch andere Bakterien ausgelöst wurde, das heißt, nicht durch solche vom Typ *Staphylococcus spp.,* kann sinnvoll sein, da- wie vorstehend ausgeführt - der Wirkstoff des Calophyllum Inophyllum Samenöls auch gegen Koagulase-negative Staphylokokken Wirkung entfaltet, die lediglich als Opportunisten am Infektionsgeschehen beteiligt sein können und nicht ursächlich für die Mastitis verantwortlich sind.

Als besonders vorteilhaft hinsichtlich dieser Wirksamkeit hat sich eine derartige Verwendung herausgestellt, bei der die pharmazeutische Formulierung 20 ppm, vorzugsweise 50 ppm, weiter vorzugsweise 100 ppm Calophyllum Inophyllum Samenöl enthält.

Alternativ ist es aber ebenso denkbar, die pharmazeutische Formulierung aus reinem Calophyllum Inophyllum Samenöl zu bilden, also einen Stoff zur Behandlung der Mastitis zu verwenden, der zu 100 % aus Calophyllum Inophyllum Samenöl besteht. Aufgrund der öligen Konsistenz des reinen Undi-Öls ist dieses durchaus praktisch anwendbar und einfach auf zu behandelnde Hautpartien applizierbar.

Ferner ist die Verwendung einer solchen pharmazeutischen Formulierung besonders von Vorteil, die neben dem eigentlichen Wirkstoff des Calophyllum Inophyllum Samenöls einen pflanzlichen Grundstoff, insbesondere eine natürliche Fettzubereitung und/oder Öle, Fette, Wachse, Vitamine, Propolis und/oder Pflanzenextrakte enthält. Als Pflanzenextrakte seien hier beispielsweise Aloe Vera, Ginseng, Kamille und Arnika genannt, weitere Pflanzenextrakte sind aber ebenso denkbar.

Im Besonderen sei hier die vorzugsweise Verwendung der genannten natürlichen Fettzubereitung als Trägerstoff für das Calophyllum Inophyllum Samenöl hervorgehoben. Unter dem Begriff "natürliche Fettzubereitung" ist in diesem Zusammenhang eine Zubereitung beispielsweise gemäß der EP 2 011 483 B1 zu verstehen. Diese besteht im Wesentlichen aus mittelkettigen und langkettigen Triglyceriden, wobei der übergroße Anteil aus einer Mischung aus mittelkettigen Triglyceriden gebildet wird (zwischen 60 M-% und 98 M-%). Eine Mischung langkettiger Triglyceride ist zu einem Anteil von 2 M-% bis 40 M-% vorgesehen. Eine auf diese Art und Weise gebildete natürliche Fettzubereitung weist optisch und rheologisch die gleichen Eigenschaften auf wie eine handelsübliche Vaseline. Im Unterschied zu einer solchen ist sie jedoch rein pflanzlich hergestellt und kommt in der Herstellung vollständig ohne Mineralölprodukte aus. Umgangssprachlich kann eine derartige natürliche Fettzubereitung auch als "pflanzliche Vaseline" bezeichnet werden.

Die im Rahmen der erfindungsgemäßen pharmazeutische Formulierung zur anspruchsgemäßen Verwendung kommt mit einem Grundstoff auf Basis einer solchen natürlichen Fettzubereitung vollständig ohne ein mineralölbasiertes Petrolatum beziehungsweise eine mineralölbasierte Vaseline aus. Hinsichtlich der Behandlung einer Mastitis - beispielsweise bei einer erkrankten Milchkuh - bietet ein solcher Grundstoff den erheblichen Vorteil, dass eine Behandlung des Euters der Milchkuh möglich ist, ohne dass eine Verunreinigung der gewonnenen Milch mit möglicherweise schädlichen Bestandteilen aus der auf Mineralöl basierenden Vaseline befürchtet werden muss. Die zugrunde liegende Aufgabe wird ferner durch eine pharmazeutische Formulierung gelöst, die Calophyllum Inophyllum Samenöl und eine natürliche Fettzubereitung enthält. Die erfindungsgemäße Verwendung ist mittels einer solchen erfindungsgemäßen pharmazeutischen Formulierung besonders vorteilhaft. Dies leitet sich in erster Linie durch die besonders sinnvolle Kombination des Wirkstoffs des Calophyllum Inophyllum Samenöls mit einer auf natürlichen Stoffen basierenden und hautfreundlichen natürlichen Fettzubereitung her. Wie vorstehend bereits erläutert wurde, eignet sich das Undi-Öl für die direkte Anwendung auf Hautoberflächen, da es die für eine typische Hautflora relevanten Bakterienarten wie Mikrokokken und Milchsäurebakterien nicht angreift. Gleichzeitig ist die Wirkung gegen die für die Mastitis hauptsächlich verantwortlichen pathogenen Staphylokokkenstämme besonders ausgeprägt. Die Kombination mit einem pflanzlichen Grundstoff in Form der genannten natürlichen Fettzubereitung oder auch "pflanzlichen Vaseline" führt schließlich zu

dem besonderen Vorteil, dass die fertige pharmazeutische Formulierung völlig frei von Schadstoffen ist, die gesundheitlich bedenklich wären. Eine solche pharmazeutische Formulierung enthält demnach weder Nebenwirkungen auslösende Wirkstoffe, insbesondere keine Antibiotika, noch schädliche Rückstände, die aus der Herstellung des Trägerstoffs beziehungsweise des Grundstoffs herrühren könnten. Eine Behandlung von Mastitis beispielsweise bei Milchvieh parallel zur Milchproduktion ist somit besonders gut möglich.

Vorteilhafterweise sollte eine solche pharmazeutische Formulierung verwendet werden, die 20 ppm bis 100.000 ppm Calophyllum Inophyllum Samenöl enthält. Versuche haben zeigen können, dass diese Einstellung der Mengenverhältnisse zu einer optimalen Kombination aus Wirksamkeit gegen pathogene Bakterien und Anwendbarkeit hinsichtlich der rheologischen Eigenschaften der Formulierung führt.

Ferner sollte die erfindungsgemäße pharmazeutische Formulierung eine antibakterielle Wirksamkeit gegen Staphylokokken, insbesondere gegen *Staphylococcus aureus,* und Streptokokken aufweisen und sich entsprechend zur Behandlung von Mastitis bei landwirtschaftlichen Nutztieren eignen.

### Ausführungsbeispiele

Die in dem erfindungsgemäßen Verfahren zur Anwendung kommende erfindungsgemäße pharmazeutische Formulierung ist beispielsweise zum überwiegenden Teil, nämlich zu 95 %, aus einer natürlichen Fettzubereitung gemäß der EP 2 011 483 B1 und im Übrigen aus 5 % Calophyllum Inophyllum Samenöl gebildet. Eine solche pharmazeutische Formulierung kann beispielsweise zur Behandlung von Mastitis bei Milchkühen eingesetzt werden.

## Patentansprüche

1. Calophyllum Inophyllum Samenöl zur Verwendung bei der Behandlung von Mastitis bei landwirtschaftlichen Nutztieren.

2. Calophyllum Inophyllum Samenöl nach Anspruch 1, **dadurch gekennzeichnet, dass** das landwirtschaftliche Nutztier eine Milchkuh ist.

3. Calophyllum Inophyllum Samenöl nach Anspruch 1 oder 2, **dadurch gekennzeichnet, dass** die Mastitis durch eine bakterielle Infektion mit Staphylokokken, Streptokokken, Klebsiella, Actinomyces, Corynebaktieren, Listerien, coliformen Erregern und/oder Pseudomonas hervorgerufen worden ist.

4. Calophyllum Inophyllum Samenöl nach einem der Ansprüche 1 bis 3, **dadurch gekennzeichnet, dass** die Mastitis durch eine Infektion mit Staphylokokken, insbesondere *Staphylococcus aureus,* und/oder Streptokokken, insbesondere *Streptococcus agalactiae,* hervorgerufen worden ist.

5. Pharmazeutische Formulierung, enthaltend Calophyllum Inophyllum Samenöl und eine natürliche Fettzubereitung, letztere enthaltend
- 60 % bis 98 % einer Mischung aus mittelkettigen Triglyceriden und
- 2 % bis 40 % einer Mischung aus langkettigen Triglyceriden.

6. Pharmazeutische Formulierung nach Anspruch 5, **dadurch gekennzeichnet, dass** sie in Form eines Öls, einer Creme, einer Salbe oder einer Emulsion vorliegt.

7. Pharmazeutische Formulierung nach Anspruch 5 oder 6, **dadurch gekennzeichnet, dass** sie mindestens 20 ppm, vorzugsweise mindestens 50 ppm, weiter vorzugsweise mindestens 100 ppm Calophyllum Inophyllum Samenöl enthält.

8. Pharmazeutische Formulierung nach einem der Ansprüche 5 bis 7, **dadurch gekennzeichnet, dass** sie Emulgatoren, Öle, Fette, Wachse, Vitamine, Propolis und/oder Pflanzenextrakte enthält.

9. Pharmazeutische Formulierung nach einem der Ansprüche 5 bis 8, **gekennzeichnet durch** eine antibakterielle Wirksamkeit gegen Staphylokokken, insbesondere gegen *Staphylococcus aureus,* und Streptokokken.

## Claims

1. Calophyllum inophyllum seed oil for use in the treatment of mastitis in farm animals.

2. Calophyllum inophyllum seed oil as claimed in claim 1, **characterized in that** the farm animal is a dairy cow.

3. Calophyllum inophyllum seed oil as claimed in claim 1 or claim 2, **characterized in that** the mastitis has been caused by a bacterial infection with staphylococcus, streptococcus, klebsiella, actinomyces, corynebacteria, listeria, coliform pathogens and/or pseudomonas.

4. Calophyllum inophyllum seed oil as claimed in one of claims 1 to 3, **characterized in that** the mastitis has been caused by an infection with staphylococcus, in particular *Staphylococcus aureus* and/or streptococcus, in particular *Streptococcus agalactiae.*

5. A pharmaceutical formulation containing Calophyllum inophyllum seed oil and a natural fat preparation, the latter containing
- 60% to 98% of a mixture of middle-chain triglycerides, and
- 2% to 40% of a mixture of long-chain triglycerides.

6. The pharmaceutical formulation as claimed in claim 5, **characterized in that** it is in the form of an oil, a cream, an ointment or an emulsion.

7. The pharmaceutical formulation as claimed in claim 5 or claim 6, **characterized in that** it contains at least 20 ppm, preferably at least 50 ppm, more preferably at least 100 ppm of Calophyllum inophyllum seed oil.

8. The pharmaceutical formulation as claimed in one of claims 5 to 7, **characterized in that** it contains emulsifying agents, oils, fats, waxes, vitamins, propolis and/or plant extracts.

9. The pharmaceutical formulation as claimed in one of claims 5 to 8, **characterized by** an antibacterial activity against staphylococci, in particular against *Staphylococcus aureus,* and streptococci.

## Revendications

1. Huile de graines de Calophyllum Inophyllum, destinée à être utilisée dans le traitement de la mammite chez les animaux d'élevage.

2. Huile de graines de Calophyllum Inophyllum selon la revendication 1, **caractérisée en ce que** l'animal d'élevage est une vache laitière.

3. Huile de graines de Calophyllum Inophyllum selon les revendications 1 ou 2, **caractérisée en ce que** la mammite est due à une infection bactérienne provoquée par des Staphylocoques, des Streptocoques, des Klebsiella, des Actinomyces, des espèces de Corynebacterium, des espèces de Listeria, des germes coliformes et/ou des Pseudomonas.

4. Huile de graines de Calophyllum Inophyllum selon l'une des revendications 1 à 3, **caractérisée en ce que** la mammite est due à une infection provoquée par des staphylocoques, notamment par *Staphylococcus aureus,* et/ou par des streptocoques, notamment par *Streptococcus agalactiae.*

5. Formulation pharmaceutique, contenant de l'huile de graines de Calophyllum Inophyllum et une préparation naturelle de lipides, cette dernière contenant
- 60 % à 98 % d'un mélange de triglycérides à chaîne moyenne, et
- 2 % à 40 % d'un mélange de triglycérides à chaîne longue.

6. Formulation pharmaceutique selon la revendication 5, **caractérisée en ce qu**'elle se présente sous forme d'une huile, d'une crème, d'une pommade ou d'une émulsion.

7. Formulation pharmaceutique selon les revendications 5 ou 6, **caractérisée en ce qu**'elle contient au moins 20 ppm, de préférence au moins 50 ppm, de manière encore préférée au moins 100 ppm d'huile de graines de Calophyllum Inophyllum.

8. Formulation pharmaceutique selon l'une des revendications 5 bis 7, **caractérisée en ce qu**'elle contient des émulsifiants, des huiles, des graisses, des cires, des vitamines, de la propolis et/ou des extraits de plantes.

9. Formulation pharmaceutique selon l'une des revendications 5 à 8, **caractérisée par** une efficacité antibactérienne contre les staphylocoques, notamment contre *Staphylococcus aureus,* et les streptocoques.
